Europäisches Patentamt

(19) European Patent Office

Office européen des brevets

(11) Veröffentlichungsnummer: **0 036 439**
**B1**

## (12) EUROPÄISCHE PATENTSCHRIFT

(45) Veröffentlichungstag der Patentschrift:
**04.04.84**

(51) Int. Cl.³: **A 61 K 7/16**, A 61 K 7/26

(21) Anmeldenummer: **80105200.2**

(22) Anmeldetag: **02.09.80**

(54) **Zahncreme mit hohem Schaumvermögen.**

(30) Priorität: **26.03.80 DE 3011618**

(43) Veröffentlichungstag der Anmeldung:
**30.09.81 Patentblatt 81/39**

(45) Bekanntmachung des Hinweises auf die Patenterteilung:
**04.04.84 Patentblatt 84/14**

(84) Benannte Vertragsstaaten:
**AT BE CH DE FR GB IT LI LU NL SE**

(56) Entgegenhaltungen:
**DE - A - 2 712 228**
**DE - A - 2 747 092**
**FR - A - 2 156 149**
**GB - A - 1 427 300**
**US - A - 3 839 590**
**US - A - 4 143 126**

(73) Patentinhaber: **Württembergische Parfümeriefabrik GmbH, Postfach 12 70, D-7332 Eislingen (DE)**

(72) Erfinder: **Scheller, Hans Ulrich, Dr.,
Schillerstrasse 21-27, D-7332 Eislingen (DE)**

(74) Vertreter: **Werner, Hans-Karsten, Dr. et al,
Deichmannhaus am Hauptbahnhof, D-5000 Köln 1 (DE)**

## Zahncreme mit hohem Schaumvermögen

Die Erfindung betrifft eine Zahncreme mit hohem Schaumvermögen mit einem Tensidgehalt von 1 bis 2,5% und 1 bis 10 Gew.-% kaltlöslicher Gelatine oder kaltlöslicher saurer Gelatinehydrolysate. Das hohe Schaumvermögen von Zahnpasten wird von einer großen Anzahl von Verbrauchern bevorzugt, da der starke Schaum auch das Gefühl intensiver Reinigung vermittelt. Von besonderer Bedeutung ist hohes Schaumvermögen für eine Zahncreme mit optischer Putzkontrolle, gemäß DE-OS 2 747 092, bei der Farbstoffe durch starkes Schäumen optisch verdeckt und somit entfärbt werden.

Das starke Schaumvermögen kann bisher nur erreicht werden durch einen hohen Gehalt an schäumenden Tensiden. Gemäß DE-OS 2 747 092 sind deshalb Tensidgehalte von mindestens 2,5% erforderlich, um überhaupt den optischen Effekt der Putzkontrolle zu erzielen. Es ist jedoch bekannt, daß höhere Konzentrationen an Netzmitteln sich ungünstig auf die geschmacklichen Eigenschaften der Zahnpasten auswirken. Dies gilt insbesondere auch für seifenhaltige Zahnpasten. Weiterhin ist bekannt, daß Zahnpasten mit höheren Netzmittelanteilen das Interzellulargefüge des Epithals der Mundschleimhaut auflockern, wobei die mechanische Scheuerwirkung der Zahnbürste den Abschilferungsvorgang noch verstärkt. In der Literatur liegen zahlreiche Hinweise auf Epithal-Läsionen durch grenzflächenaktive Stoffe vor.

Aus der DE-OS 2 712 228 ist bekannt, Eiweißhydrolysat aus kollagenhaltigen Materialien als Zusatz zu Waschmitteln und Zahnpflegemitteln zu verwenden. Durch diesen Zusatz soll die Pflege der Zähne und des Zahnfleisches verbessert werden. Das Eiweißhydrolysat soll eine durchblutungsfördernde Wirkung auf das Zahnfleisch haben und es dadurch festigen. In einem der Beispiele sind zwar ca. 1% medizinisches Seifenpulver und 0,7% laurylsulfosaures Natrium als Zusatz erwähnt. Es fehlen jedoch jegliche Hinweise auf starkes oder stärkeres Schaumvermögen. Es fehlt somit jeglicher Hinweis darauf, daß die Kombination aus Tensiden und kaltlöslicher Gelatine oder kaltlöslicher saurer Gelatinehydrolysate geeignet ist, ein so hohes Schaumvermögen zu erzielen, daß auf dieser Basis eine Zahncreme mit Putzkontrolle hergestellt werden kann.

Die Erfindung hat sich die Aufgabe gestellt, eine neue Zahncreme mit hohem Schaumvermögen zu entwickeln, bei dem der Tensidgehalt nur 1 bis 2,5 Gew.-% beträgt und trotzdem eine optische Putzkontrolle gemäß DE-OS 2 747 092 möglich ist. Erreicht wird dies durch einen Zusatz von 1 bis 10 Gew.-% kaltlöslicher Gelatine oder kaltlöslicher saurer Gelatinehydrolysate. Gegenstand der vorliegenden Erfindung ist somit eine Zahncreme mit hohem Schaumvermögen mit einem Tensidgehalt von 1 bis 2,5% und 1 bis 10 Gew.-% kaltlöslicher Gelatine oder kaltlöslicher saurer Gelatinehydrolysate, die dadurch gekennzeichnet ist, daß sie eine ausreichende Menge eines Farbstoffes enthält, und bei intensivem nicht unterbrochenem Putzen der Zähne mit der Zahnbürste der zu Beginn des Putzvorganges farbige Schaum des Zahnpflegemittels nicht vor Ablauf von 20 oder mehr Sekunden eine Aufhellung um eine Helligkeitsdifferenz, $\Delta$ L im Bereich von 10 bis 30, vorzugsweise 15 bis 25, aufweist (wobei die Helligkeit L = 0 schwarz und L = 100 weiß entspricht) und zum weißen oder nur schwach gefärbten Schaum entfärbt ist.

Dieses Ergebnis war nicht vorhersehbar, da bekannt ist, daß Tenside und gelatineartige Eiweißprodukte miteinander zu Komplexen reagieren, welche eine Reihe typischer Eigenschaften der beiden Ausgangskomponenten nicht mehr oder nur noch in sehr abgeschwächter Form aufweisen. Insbesondere die Eigenschaft der Tenside zu benetzen und zu schäumen wird durch die Anlagerung an Proteinstrukturen erheblich vermindert. Es war daher nicht vorherzusehen, daß die Kombination aus Tensiden und Gelatine oder gelatineartigen Eiweißprodukten in Zahncreme dennoch zu einem hohen Schaumvermögen führen würden. Dieser Vorteil ist erfindungsgemäß von besonderer Bedeutung bei einer Zahncreme, gemäß DE-OS 2 747 092, da die optische Putzkontrolle nur durch den Zusatz von Tensidmengen oberhalb von 2,5% möglich war, obwohl geschmacklich als auch zahnmedizinisch der Wunsch bestand, den Tensidgehalt nicht höher als 1 bis 2% zu wählen.

Als Gelatine oder gelatineartige Eiweißprodukte eignen sich erfindungsgemäß alle lebensmittelchemisch und physiologisch geeigneten Qualitäten an Gelatine und Gelatine- bzw. Kollagenhydrolysaten. Besonders geeignet sind Qualitäten, die in kaltem Wasser löslich sind und obendrein ein verstärktes eigenes Schaumvermögen aufweisen. Zu dieser Gruppe zählen die sauer extrahierten Gelatinequalitäten aus Schweineschwarten. Weitere bevorzugte Qualitäten sind die Gelatinetypen Kupfer und Kupfer A/B, sowie das Gelatinehydrolysat CF der Deutschen Gelatinefabriken Stoess & Co. GmbH, Eberbach/Baden. Diese Qualitäten haben nur noch eine geringe Viskosität und Gelierfähigkeit, einen niedrigen pH-Wert, sind in kaltem Wasser löslich und weisen ein relativ hohes eigenes Schaumvermögen auf. Es ist zwar in der Literatur schon vorgeschlagen worden, Zahnpasten mit Hilfe von Eiweißstoffen, insbesondere Gelatine zu verdicken. Praktisch ist dieser Weg jedoch nie durchgeführt worden, da er mit erheblichen technischen Schwierigkeiten verbunden ist. Herkömmliche Gelatine muß nämlich heiß gelöst werden und verzögert dadurch den Produktionsprozeß. Weiterhin ist eine derartige Zahncreme temperaturlabil und somit für die Praxis ungeeignet. Die erfindungsgemäß eingesetzten Gelatinemengen sind wesentlich niedriger als für eine Verdickung notwendig wäre. Sie beeinflussen daher die Viskosität der Zahncreme nicht oder so unwesentlich, daß keine Störungen beobachtet werden.

Überraschenderweise vermindert die erfindungsgemäß zugesetzte Gelatine die negativen Einflüsse der Netzmittel auf die Mundschleimhaut. Weiterhin wird der negative Einfluß der Netzmittel auf den Geschmack der Zahncreme durch den erfindungsgemäßen Zusatz der Gelatine reduziert. Der relativ häufig subjektiv festgestellte Effekt der Mundtrockenheit in Folge hohem Netzmittelgehaltes wird bei der erfindungsgemäßen Zahncreme nicht mehr wahrgenommen.

Bei einer Zahncreme mit Putzkontrolle gemäß DE-OS 2 747 092 ist es erfindungsgemäß ohne weiteres möglich, den Gehalt an Tensiden auf 1,5 bis 2% zu senken und trotzdem den gleichen Entfärbungseffekt durch starke Schaumbildung zu bewirken.

Als Tenside eignen sich erfindungsgemäß besonders wasserlösliche Salze von höheren Alkylsulfaten, beispielsweise Natriumlaurylsulfat, aliphatische Acylamide, gesättigte Monoaminocarbonsäuren, vorzugsweise Natrium-N-Lauroyl-Sarcosinat, Taurin-Fettsäure-Amide, Salze von sulfonierten Monogliceriden höherer Fettsäuren, Fettsäureester der Isäthionsäure und deren Salze, nichtionischer Tenside, wie Alkylenoxidkondensate mit Fettalkohol und ein- oder mehrwertige Amine, Zuckerester, langkettige Aminoxide, ampholytische Oxide, beispielsweise Betain oder langkettige Alkylamino-Carbonsäuren und kationaktive Tenside, beispielsweise quartäre Ammoniumverbindungen. Diese Substanzen können alleine oder als Gemische verwendet werden und liegen erfindungsgemäß in Mengen von 1 bis 2,5, vorzugsweise 1,5 bis 2 Gew.-% vor. Niedrigere Konzentrationen liefern ein nicht ausreichendes Schaumvermögen, höhere Konzentrationen sind aus obengenannten Gründen unerwünscht.

Die Gelatine und gelatineartigen Eiweißprodukte liegen in Mengen von 1 bis 10, vorzugsweise 2 bis 7 Gew.-% vor. Niedrigere Mengen tragen ungenügend zur Schaumbildung bei, so daß der Tensidgehalt unnötig erhöht werden müßte. Höhere Gehalte als 10% sind unerwünscht, weil dann eine merkliche Beeinflussung der Viskosität zu beobachten ist, die bei der Thermolabilität der Gelatine zu unerwünschten Effekten führt. Die erfindungsgemäße Zahncreme kann die üblichen Zusätze und Aufbaustoffe enthalten. Als Feuchthaltemittel finden Glycerin, Polyglykole mit niedrigem Molekulargewicht und Zuckeralkohole, wie Sorbit, Mannit und Xylit Verwendung. Als Verdickungsmittel eignen sich vorzugsweise die Alkalisalze der Carboxymethylcellulose insbesondere Natriumcarboxymethylcellulose, Hydroxyalkylcellulose, insbesondere Hydroxyäthylcellulose, Pflanzengummen, wie Traganth, Gummi arabicum, Caraya Gummi und Irish Moos, synthetische Polyelektrolyte und gegebenenfalls auch anorganische Verdickungsmittel, beispielsweise kolloidale Magnesiumaluminiumsilikate. Besonders bevorzugt wird pyrogene Kieselsäure. Weiterhin erhält die erfindungsgemäße Zahncreme Calciumcarbonat oder Dicalciumphosphat sowie üblicherweise Aroma- und Geschmacksstoffe, Konservierungsmittel etc. Bevorzugte Ausführungsformen der Erfindung enthalten Fluorverbindungen in einer solchen Menge, daß die Konzentration an reinem Fluor zwischen 0,01 bis 1 Gew.-%, vorzugsweise 0,1 bis 0,5 Gew.-% beträgt.

Geeignete Fluor-Verbindungen sind insbesondere die verschiedenen Salze der Monofluor-Phosphorsäure sowie die verschiedenen das Fluor in ionischer Form enthaltenden Fluoride, insbesondere Alkalifluoride, wie Natrium-, Lithium-, Kalium-, Ammoniumfluorid, Zinnfluorid, Manganfluorid, Zirkonium- und Aluminiumfluorid, sowie Gemische oder Anlagerungsprodukte dieser Fluoride untereinander oder mit anderen Fluorverbindungen, beispielsweise Kalium- oder Natriummanganfluorid. Weitere erfindungsgemäß einsetzbare Fluoride sind beispielsweise Zinkfluorid, Germaniumfluorid, Palladiumfluorid, Titanfluorid, Alkalifluorzirkonate und Fluorsulphate. Ebenso können mit Erfolg die bekannten Additionsprodukte aus langkettigen Aminosäuren und Fluorwasserstoff, Monoäthanol-Aminohydrofluorid und Methyltriäthylammoniumfluorid eingesetzt werden.

Die erfindungsgemäße Zahncreme kann weiterhin zahnsteinlösende Substanzen wie die Phosphonsäuren sowie Bisbiguanide wie Chlorhexidin, Fluorhexidin und Alexidin sowie deren wasserlösliche Salze enthalten.

Eine Zahncreme mit optischer Putzkontrolle gemäß DE-OS 2 747 092 kann prinzipiell alle dort beschriebenen Bestandteile enthalten und wird vorzugsweise nach den dort bevorzugt genannten Weisen hergestellt, mit der einzigen Abweichung, daß der Tensidgehalt gesenkt wird stattdessen der erfindungsgemäße Anteil Gelatine oder gelatineartige Eiweißprodukte zugefügt wird.

In den nachfolgenden Beispielen sind typische Ausführungsformen der neuen Zahncreme beschrieben.

### Beispiel 1

Zahncreme hergestellt unter Verwendung einer Gelatine herkömmlicher Lebensmittelqualität (Gelatine Cu der Deutschen Gelatinefabriken Stoess & Co. GmbH, Eberbach/Baden).

| Substanz | Menge in Gew.-% |
|---|---|
| Carboxymethylcellulose | 0,8 |
| Calciumcarbonat | 25,0 |
| Laurylalkoholsulfat-Na | 2,0 |

Fortsetzung

| Substanz | Menge in Gew.-% |
|---|---|
| Myristinsäuretaurid-Na | 0,5 |
| Gelatine Cu | 3,0 |
| Glycerin 86% oder Sorbit | 12,0 |
| p-Hydroxybenzoesäure-methylester-Na | 0,2 |
| Pyrogene Kieselsäure | 2,0 |
| Aromastoffe | 2,0 |
| L-Blue Nr. 3 | 0,006 |
| CI Nr. 42 051 | |
| Wasser, demineralis | ad 100,0 |

Die Wirkung der erfindungsgemäßen Zahncreme hinsichtlich der Entfärbung des gebildeten Schaumes wird durch Versuche von 5 Personen mit einer elektrischen Zahnbürste (AEG-Prinzeß) getestet. Es wurden jeweils 2 cm Pastenstrang (entsprechen 0,5 g) auf die angefeuchtete Zahnbürste aufgetragen und der Putzvorgang solange fortgesetzt, bis der zu Beginn deutlich blau eingefärbte Schaum weiß war oder aber innerhalb einer sinnvollen Putzzeit keine weitere Farbveränderung mehr zeigte. Die Ergebnisse sind in der Tabelle 1 zusammengestellt.

Tabelle 1

| 1. Testperson | nach 100 Sec. weiß |
|---|---|
| 2. Testperson | nach 100 Sec. weiß |
| 3. Testperson | nach  80 Sec. weiß |
| 4. Testperson | nach  90 Sec. weiß |
| 5. Testperson | nach  80 Sec. weiß |

durchschnittliche Dauer =    90 Sec.

Zum Vergleich wurde eine Zahncreme gemäß DE-OS 2 747 092 getestet mit folgender Rezeptur.

| Substanz | Menge in Gew.-% |
|---|---|
| Carboxymethylcellulose | 1,0 |
| Calciumcarbonat | 25,0 |
| Laurylalkoholsulfat-Na | 3,0 |
| Myristinsäuretaurid-Na | 0,5 |
| Glycerin 86% oder Sorbit | 12,0 |

| Substanz | Menge in Gew.-% |
|---|---|
| p-Hydroxybenzoesäure-methylester-Na | 0,2 |
| Pyrogene Kieselsäure | 2,0 |
| Aromastoffe | 2,0 |
| L-Blue Nr. 3 | 0,006 |
| CI Nr. 42 051 | |
| Wasser, demineralis | ad 100,0 |

Diese Zahncreme enthielt 3,5 Gew.-% Tenside. Die Putzversuche wurden wie im Beispiel 1 durchgeführt und hatten folgende Ergebnisse:

| 1. Testperson | nach 110 Sec. weiß |
|---|---|
| 2. Testperson | nach  80 Sec. weiß |
| 3. Testperson | nach  90 Sec. weiß |
| 4. Testperson | nach 110 Sec. weiß |
| 5. Testperson | 90 Sec. weiß |

durchschnittliche Dauer =    96 Sec.

Aus diesen Versuchen geht eindeutig hervor, daß der Gesamttensidanteil gegenüber der bekannten Rezeptur um 1 Gew.-% reduziert werden konnte, und trotzdem annähernd identische Ergebnisse erzielt werden.

Beispiel 2

Zahncreme mit saurer extrahierter Gelatine aus Schweineschwarten.

| Substanz | Menge in Gew.-% |
|---|---|
| Carboxymethylcellulose | 1,0 |
| Calciumcarbonat | 25,0 |
| Laurylalkoholsulfat-Na | 1,5 |
| Myristinsäuretaurid-Na | 0,5 |
| Glycerin 86% oder Sorbit | 12,0 |
| p-Hydroxybenzoesäure-methylester-Na | 0,2 |
| Pyrogene Kieselsäure | 2,0 |
| Aromastoffe | 2,0 |
| Gelatinehydrolysat | 3,5 |

Fortsetzung

| Substanz | Menge in Gew.-% |
|---|---|
| L-Blue Nr. 3 | 0,006 |
| CI Nr. 42 051 | |
| Wasser, demineralis | ad 100,0 |

Die Putzversuche führten zu folgenden Ergebnissen:

| 1. Testperson | nach 120 Sec. weiß |
|---|---|
| 2. Testperson | nach 100 Sec. weiß |
| 3. Testperson | nach 80 Sec. weiß |
| 4. Testperson | nach 90 Sec. weiß |
| 5. Testperson | nach 90 Sec. weiß |

durchschnittliche Dauer =    96 Sec.

Im Vergleich zur bekannten Rezeptur ist der Tensidgehalt dieser Zahncreme auf 2,0% gesenkt und trotzdem werden völlig vergleichbare Ergebnisse erzielt.

Bei dem Gelatinehydrolysat handelt es sich um eine kalt wasserlösliche, stark schäumende Qualität der Deutschen Gelatinefabriken Stoess & Co. GmbH, Eberbach (Gelatinehydrolysat CF).

## Patentanspruch

Zahncreme mit hohem Schaumvermögen mit einem Tensidgehalt von 1 bis 2,5% und 1 bis 10 Gew.-% kaltlöslicher Gelatine oder kaltlöslicher saurer Gelatinehydrolysate, dadurch gekennzeichnet, daß sie eine ausreichende Menge eines Farbstoffes enthält, und bei intensivem nicht unterbrochenem Putzen der Zähne mit der Zahnbürste der zu Beginn des Putzvorganges farbige Schaum des Zahnpflegemittels nicht vor Ablauf von 20 oder mehr Sekunden eine Aufhellung um eine Helligkeitsdifferenz, $\Delta$ L im Bereich von 10 bis 30, vorzugsweise 15 bis 25, aufweist (wobei die Helligkeit L = 0 schwarz und L = 100 weiß entspricht) und zum weißen oder nur schwach gefärbten Schaum entfärbt ist.

## Claim

Dentifrice with high foaming ability having a tenside content of from 1 to 2.5% and containing 1 to 10% by weight of cold-soluble gelatin or cold-soluble gelatin hydrolyzates, characterized in that it contains a sufficient amount of a colorant and that, upon intense uninterrupted cleaning the teeth by using a toothbrush, the colored dentifrice foam produced at the beginning of brushing shows decolorization by a brightness difference $\Delta$ L in the range of 10 to 30, and preferably from 15 to 25, not before a period of 20 or more seconds has passed (the brightnesses L = 0 corresponding to black and L = 100 corresponding to white) and is decolorized to a white or a faintly colored foam.

## Revendication

Pâte dentifrice à haut pouvoir moussant, avec une teneur en tensioactifs entre l et 2,5% et avec 1 à 10% en poids d'une gélatine soluble à froid ou d'un hydrolysat de gélatine acide soluble à froid, caractérisée en ce qu'elle contient une quantité suffisante d'un colorant, et que, lors d'un nettoyage intensif ininterrompu des dents avec la brosse à dents, la mousse du produit d'hygiène dentaire, colorée au début de l'opération de nettoyage, présente, au plus tôt après écoulement d'au moins 20 secondes, une augmentation de luminosité correspondant à une différence de luminosité $\Delta$ L dans la plage de 10 à 30, de préférence de 15 à 25 (où la luminosité L = 0 correspond au noir et L = 100 correspond au blanc), et se décolore en mouse blanche ou seulement faiblement colorée.